# EUROPEAN PATENT SPECIFICATION

(11) **EP 2 077 982 B1**
(45) Date of publication and mention of the grant of the patent: **14.08.2013**
(21) Application number: 07863624.8
(22) Date of filing: 29.10.2007
(51) Int. Cl.: C07C 2/00, C07C 2/66, C07C 15/02

(54) **Process for producing phenylalkanes of desired 2-phenyl content**
Verfahren zur Herstellung von Phenylalkanen mit gewünschtem 2-Phenylgehalt
Procédé de production de phénylalcanes de la teneur en 2-phényle souhaitée

(30) Priority: 30.10.2006 US 863459 P
(43) Date of publication of application: 15.07.2009
(73) Proprietor: UOP LLC, Des Plaines, IL 60017-5017 (US)
(72) Inventor: RILEY, Mark G., Des Plaines, IL 60017-5017 (US); SOHN, Stephen W., Des Plaines, Illinois 60017-5017 (US)
(74) Representative: Hayes, Adrian Chetwynd
(86) International application number: PCT/US2007/082899
(87) International publication number: WO 2008/055121

(56) References cited:
- US-A1- 2005 010 072
- US-A1- 2006 224 031

## Description

### BACKGROUND OF THE INVENTION

The alkylation of benzene with olefins is a widely practice process, especially for the production of alkylbenzenes. Alkylbenzenes having alkyl groups of 8 to 14 carbon atoms per alkyl group, for instance, are commonly sulfonated to make surfactants.

Various processes have been proposed to alkylate benzene. One commercial process involves the use of hydrogen fluoride as the alkylation catalyst. The use and handling of hydrogen fluoride does provide operational concerns due to its toxicity, corrosiveness and waste disposal needs. Solid catalytic processes have been developed that obviate the need to use hydrogen fluoride. Improvements in these solid catalytic processes are sought to further enhance their attractiveness through reducing energy costs and improving selectivity of conversion while still providing an alkylbenzene of a quality acceptable for downstream use such as sulfonation to make surfactants.

Alkylbenzenes, to be desirable for making sulfonated surfactants must be capable of providing a sulfonated product of suitable clarity, biodegradability and efficacy. With respect to efficacy, alkylbenzenes having higher 2-phenyl contents are desired as they tend, when sulfonated, to provide surfactants having better solubility and detergency. Thus, alkylbenzenes having a 2-phenyl isomer content in the range from 30 to 40 percent are particularly desired.

Heretofore, numerous proposals have been made for solid catalysts for the alkylation of benzene. US Application Publication No. 2005/0010072A1 provides a list of some of the solid catalysts that have been proposed and the relative 2-phenyl contents of alkylbenzenes made using the catalysts.

Achieving a desired 2-phenyl content will not necessarily result in a commercially viable process. The process must have suitable selectivities to the mono-alkylbenzenes. Under alkylation conditions an alkylbenzene or an olefin can react with another olefin to produce heavies. One technique to minimize the formation of heavies is to operate at higher benzene to olefin mole ratios such that by relative concentrations, the reaction of an olefin with benzene is favored as compared to an alkylbenzene or another olefin. However, the additional benzene must be recovered from the alkylbenzene product, commonly by distillation, resulting in incrementally increased energy costs.

US 6,133,492 discloses a process for the alkylation of benzene using a mixed catalyst. The catalyst comprises a fluorine-containing mordenite and a solid alkylation catalyst such as aluminum chloride, fluorine-containing clay, or silica-alumina catalyst.

US 6,521,804B1 discloses benzene alkylation with a mordenite catalyst and a silica-alumina catalyst.

US 6,977,319B2 discloses a process for making alkylated aromatics using a catalyst compositions comprising zeolite Y and mordenite zeolite having a controlled macropore structure.

US Application Publication 2005/0010072A1 discloses an alkylation process using at least two catalysts in at least two distinct reaction zones. A preferred process uses Y zeolite in one reaction zone and mordenite in the other zone.

US 6,756,030B1 discloses a family of molecular sieves identified as UZM-8. At column 7, lines 44 to 52, the use of UZM-8 for alkylation of aromatics is disclosed.

Gong, et al., in Catalytic Performance of Nanometer MCM-49 Zeolite for Alkylation Reaction of Benzene with 1-Dodecene, Chinese Journal of Catalysis, Vol. 25, No. 10, 809-813, October 2004, relate increased activity with high selectivity for 2- and 3-phenylalkanes using MCM-49, a structurally similar molecular sieve to UZM-8, having a diameter of 300 to 500 nanometers and a thickness of 20 to 25 nanometers.

### SUMMARY OF THE INVENTION

By this invention processes are provided for the alkylation of benzene with acyclic mono-olefin, especially with olefin of from 8 to 16 carbon atoms per molecule, to produce phenylalkanes, which processes provide a product having desirable 2-phenyl content. In the preferred aspects of this invention, a low aromatic to mono-olefin mole ratio can be used without undue production of heavies, thereby enhancing the economic attractiveness of a solid catalyst alkylation process. The processes of this invention are based upon the use of a combination of acidic FAU molecular sieve and UZM-8 molecular sieve as catalytically active materials.

Advantageously the 2-phenyl content provided by the processes of this invention remains constant over the lifetime of the catalyst even though the catalysts undergo deactivation, and regeneration. Typically solid catalysts require frequent regenerations, and thus the ability to have a balance between the catalytic properties of the molecular sieves during deactivation and after regeneration provided by this invention facilitates commercial processes for making phenylalkanes of a consistent, sought 2-phenyl content.

In its broad aspect, the processes for producing a phenylalkane product comprising contacting at least one aromatic compound, wherein the aromatic compound is benzene, and at least one acyclic mono-olefin with solid catalyst under alkylation conditions wherein at least a portion of said aromatic compound and at least a portion of said mono-olefin are contacted with a catalyst comprising acidic FAU molecular sieve and at least a portion of said aromatic compound and at least a portion of said mono-olefin are contacted with a catalyst comprising UZM-8 molecular sieve to provide a phenylalkane product having a 2-phenyl content of between 25 and 40 mass percent (based upon total phenylalkane). The catalysts may be in distinct reaction zones or may be in the same reaction zone. Preferably, the 2-phenyl content is between 26 and 36 mass percent. The selection of the 2-phenyl content may, for instance, be based on a desire to match the 2-phenyl existing alkylbenzene products such that the user need no change formulations containing or processes using the alkylbenzenes. For detergent production, 2-phenyl content of between 26 and 30 mass percent may be consistent with available alkylbenzene products. On the other hand, improved solubility may be obtained with 2-phenyl content in the range of 30 to 35 mass percent. In the preferred aspects of this invention, the alkylation conditions are sufficient to provide a phenylalkane linearity of at least 89, and sometimes at least 92, mass percent. In other preferred aspects of this invention, the alkylation conditions are sufficient that the phenylalkane product contains less than 6, preferably less than 4, and more preferably less than 3, mass percent heavies based on the mass of the total phenylalkanes.

The catalysts of this invention comprise 25 to 95 mass percent FAU molecular sieve and 5 to 75 mass percent UZM-8 molecular sieve based upon total molecular sieve in the catalyst.

### DETAIL DISCUSSION OF THE INVENTION

### The Feed and Products:

Olefin-containing aliphatic compound and aromatic compound are used for the alkylation process. The selection of the olefin and aromatic compounds is dependent upon the sought alkylation product.

The olefin-containing aliphatic compound is preferably of 6 to 40, often 8 to 28, and for detergent applications, 9 to 16, carbon atoms per molecule. The olefin-containing aliphatic compound is an acyclic, mono-olefinic compound. The positioning of the olefinic bond in the molecule is not critical as most alkylation catalysts have been found to promote migration of the olefinic bond. However, the branching of the hydrocarbon backbone is often more of a concern as the structural configuration of the alkyl group on the arylalkane product can affect performance especially in surfactant applications and for biodegradation properties. For instance, where arylalkanes are sulfonated to produce surfactants, undue branching can adversely affect the biodegradability of the surfactant. On the other hand, some branching may be desired such as the lightly branched modified alkylbenzenes such as described in US 6,187,981B1. The olefin may be unbranched or lightly branched, which as used herein, refers to an olefin having three or four primary carbon atoms and for which none of the remaining carbon atoms are quaternary carbon atoms. A primary carbon atom is a carbon atom which, although perhaps bonded also to other atoms besides carbon, is bonded to only one carbon atom. A quaternary carbon atom is a carbon atom that is bonded to four other carbon atoms. Although branched, these alkylbenzenes have been characterized by their 2-phenyl content, see for instance, US 6,589,927B1.

The olefin-containing aliphatic compound is usually a mixture of two or more olefins. For commercial processes, the feedstocks may include other components as well. These other components may comprise paraffins of 6 to 40, often 8 to 28, and for detergent applications, 9 to 16, carbon atoms per molecule. For instance, the olefin may be obtained by the dehydrogenation of a paraffinic feedstock. See, for instance, US 6,670,516B1.
Generally, for the olefin-containing feedstock, the feedstock comprises at least 10 mole percent olefin.

The source of the paraffinic feedstock is not critical although certain sources of paraffinic feedstocks will likely result in the impurities being present. Conventionally, kerosene fractions produced in petroleum refineries either by crude oil fractionation or by conversion processes therefore form suitable feed mixture precursors. Fractions recovered from crude oil by fractionation will typically require hydrotreating for removal of sulfur and/or nitrogen prior to being fed to the subject process. The boiling point range of the kerosene fraction can be adjusted by prefractionation to adjust the carbon number range of the paraffins. In an extreme case the boiling point range can be limited such that only paraffins of a single carbon number predominate. Kerosene fractions contain a very large number of different hydrocarbons and the feed mixture to the subject process can therefore contain 200 or more different compounds.

The paraffinic feedstock may be at least in part derived from oligomerization or alkylation reactions. Such feed mixture preparation methods are inherently imprecise and produce a mixture of compounds. The feed mixtures to the process may contain quantities of paraffins having multiple branches and paraffins having multiple carbon atoms in the branches, cycloparaffins, branched cycloparaffins, or other compounds having boiling points relatively close to the desired compound isomer. The feed mixtures to the process of this invention can also contain aromatic hydrocarbons.

Another source of paraffins is in condensate from gas wells. Usually insufficient quantities of such condensate are available to be the exclusive source of paraffinic feedstock. However, its use to supplement other paraffinic feedstocks can be desirable. Typically these condensates contain sulfur compounds, which have restricted their use in the past. As this invention enables the use of sulfur-containing feeds, these condensates can be used to supply paraffins for alkylation.

Paraffins may also be produced from synthesis gas (Syngas), hydrogen and carbon monoxide. This process is generally referred to as the Fischer-Tropsch process. Syngas may be made from various raw materials including natural gas and coal, thus making it an attractive source of paraffinic feedstock where petroleum distillates are not available. The Fischer-Tropsch process is a catalytic process conducted under elevated temperature and pressure. The reaction is temperature sensitive, and temperature control is essential to achieve a desired hydrocarbon product. The products from the Fischer-Tropsch process include not only paraffins but also monoolefins, diolefins, aromatics and oxygenates such as alcohols, ethers, aldehydes and ketones, and thus are normally treated to eliminate oxygenates.

The olefin-containing feedstock should be sufficiently free of impurities that can unduly adversely affect the life of the alkylation catalyst.

The aromatic-containing feedstock to the subject process comprises an aromatic or a phenyl compound, which is benzene when the process is detergent alkylation.

### The Catalysts:

In accordance with the broad aspects of the processes of this invention at least a portion of the aromatic compound and mono-olefin is contacted with a catalyst comprising acidic FAU molecular sieve and at least a portion of the aromatic compound and mono-olefin is contacted with a catalyst comprising UZM-8 molecular sieve to provide a phenylalkane product having a 2-phenyl content of between 25 and 40 mass percent.

The acidic FAU molecule sieve and the UZM-8 molecular sieve may be incorporated into the same catalyst structure or may be contained in separate catalyst structures. Preferably the acidic FAU molecular sieve has an acidity of at least 0.10, preferably at least 0.12, and sometimes at least 0.2, millimole of ammonia per gram of dry FAU molecular sieve as determined by ammonia temperature programmed desorption (ammonia TPD). In general, many larger crystal FAU molecular sieves require higher acidities than smaller crystal FAU molecular sieve to achieve similar activities. The ammonia TPD process is performed at ambient pressure and involves first heating a sample (approximately 250 milligrams) of FAU at a rate of 5°C per minute to a temperature of 550°C in the presence of an 20 volume percent oxygen in helium atmosphere (flow rate of approximately 100 milliliters per minute). After a hold of approximately one hour, helium is used to flush the system (approximately 15 minutes) and the sample is cooled to 150°C. The sample is then saturated with pulses of ammonia in helium at approximately 40 milliliters per minute. The total amount of ammonia used is greatly in excess of the amount required to saturate all the acid sites on the sample. The sample is purged with helium (40 milliliters per minute) for 8 hours to remove physisorbed ammonia. With the helium purge continuing, the temperature is increased at a rate of 10°C per minute to a final temperature of 600°C. The amount of ammonia desorbed is monitored using a calibrated thermal conductivity detector. The total amount of ammonia is found by integration. Dividing the total amount of ammonia by the dry weight of the sample yields the acidity expressed as millimoles of ammonia per gram of dry sample. The dry weight of the molecular sieve can be determined by heating the molecular sieve in flowing nitrogen at 500°C for 2 hours.

The preferred FAU molecular sieves include zeolite Y, dealuminated zeolite Y and zeolite X, including rare earth exchanged zeolites Y and X, especially zeolite Y, having a framework silica to alumina molar ratio of between 4: to 70:1, more preferably 5:1 to 30:1. The FAU is often in a hydrogen form. The FAU zeolite framework type is described in Ch. Baerlocher, W.M. Meier and D.H. Olson, "Atlas of Zeolite Framework Types," 5th ed., Elsevier: Amsterdam, 2001.

The UZM-8 molecular sieve is described in US 6,756,030B1. The UZM-8 molecular sieve is often in the hydrogen form when used as a catalyst. Where the UZM-8 is in the ammonium form, calcination of the UZM-8 can provide the hydrogen form. If the UZM-8 is in a metal cation form, exchange with ammonium cation followed by calcination can conveniently generate the hydrogen form molecular sieve.

The FAU and UZM-8 molecular sieves may have any convenient crystal size. Often the crystal sizes for FAU molecular sieve range upwards of 5 microns or more in major dimension, say, 50 to 5000, nanometers in major dimension. Crystal sizes in the lower portion of the range are sometimes preferred as the coproduction of heavies may be reduced. Major crystal dimensions of less than 500, e.g., from 50 to 300, nanometers are often desirable. See, for instance, Koegler, et al., US Application Publication No. 2003/0147805A1. Crystal size is measured visually by scanning electron microscope (SEM).

The catalyst contains a catalytically-effective amount of molecular sieve. The catalyst may contain suitable binder or matrix material such as inorganic oxides and other suitable materials. The relative proportion of molecular sieve in the catalyst may range from 10 to 99 mass-percent, with 20 to 90 mass-percent being preferred. Where a single catalyst contains both the FAU and UZM-8 molecular sieves, the mass ratio of FAU to UZM-8 molecular sieve is between 10:1 to 1:3 and preferably between 8:1 to 1:2.

A refractory binder or matrix is typically used to facilitate fabrication of the catalyst, provide strength and reduce fabrication costs. The binder should be uniform in composition and relatively refractory to the conditions used in the process. Suitable binders include inorganic oxides such as one or more of alumina, magnesia, zirconia, chromia, titania, boria and silica. The catalyst also may contain, without so limiting the composite, one or more of (1) other inorganic oxides including, but not limited to, beryllia, germania, vanadia, tin oxide, zinc oxide, iron oxide and cobalt oxide; (2) non-zeolitic molecular sieves, such as the aluminophosphates of US Pat. No. 4,310,440, the silicoaluminophosphates of US 4,440,871 and ELAPSOs of US Pat. No. 4,793,984; and (3) spinels such as MgAl2O4, FeAl2O4, ZnAl2O4, CaAl2O4, and other like compounds having the formula MO-Al2O3 where M is a metal having a valence of 2; which components can be added to the composite at any suitable point.

The catalyst may be prepared in any suitable manner. One method for preparation involves combining the binder and molecular sieve in a hydrosol and then gelling the mixture. One method of gelling involves combining a gelling agent with the mixture and then dispersing the resultant combined mixture into an oil bath or tower which has been heated to elevated temperatures such that gelation occurs with the formation of spheroidal particles. The gelling agents which may be used in this process are hexamethylene tetraamine, urea or mixtures thereof. The gelling agents release ammonia at the elevated temperatures which sets or converts the hydrosol spheres into hydrogel spheres. The spheres are then continuously withdrawn from the oil bath and typically subjected to specific aging and drying treatments in oil and in ammoniacal solution to further improve their physical characteristics. The resulting aged and gelled particles are then washed and dried at a relatively low temperature of 100° to 150°C and subjected to a calcination procedure at a temperature of 450° to 700°C for a period of 1 to 20 hours.

The combined mixture preferably is dispersed into the oil bath in the form of droplets from a nozzle, orifice or rotating disk. Alternatively, the particles may be formed by spray-drying of the mixture at a temperature of from 425° to 760°C. In any event, conditions and equipment should be selected to obtain small spherical particles; the particles preferably should have an average diameter of less than 5.0 mm, more preferably from 0.2 to 3 mm, and optimally from 0.3 to 2 mm.

Alternatively, the catalyst may be an extrudate. A multitude of different extrudate shapes are possible, including, but not limited to, cylinders, cloverleaf, dumbbell and symmetrical and asymmetrical polylobates. Typical diameters of extrudates are 1.6 mm (1/16 in.) and 3.2 mm (1/8 in.). The extrudates may be further shaped to any desired form, such as spheres, by any means known to the art.

The catalyst of the present invention may contain a halogen component, e.g., 0.1 to 4 mass percent halogen. A suitable halogen is, for example, fluoride. Frequently the catalyst need not contain any added halogen other than that associated with other catalyst components to provide the sought alkylation activity.

The catalytic composite optimally is subjected to steaming to tailor its acid activity. The steaming may be effected at any stage of the zeolite treatment. Alternatively or in addition to the steaming, the composite may be washed with one or more of a solution of ammonium nitrate; a mineral acid such as hydrogen chloride, sulfuric acid or nitric acid; and/or water.

If desired, the catalytic composite usually is dried and then calcined, e.g., at a temperature of from 400° to 600° C. in an air atmosphere for a period of from 0.1 to 10 hours.

### The Processes:

The acyclic mono-olefin is reacted with aromatic compound to produce arylalkane, or phenylalkane. Preferably, the aromatic compound is alkylated with a single mono-olefin.

The catalysts may be in the same reaction zone or in distinct reaction zones, either in parallel or in series. Thus, the processes of this invention provide for a wide variety of configurations. Each configuration may be operated differently to achieve the sought phenylalkane product.

Where the catalysts are contained in the same reaction zone, the catalysts may be blended or a single catalyst having both the FAU and UZM-8 components may be used. The ratio of the FAU and UZM-8 molecular sieves will define the 2-phenylalkane concentration in the product. Typically, as the proportion of the UZM-8 increases, so does the 2-phenylalkane concentration of the product.

Where the catalysts are contained in distinct reaction zones in series, the FAU molecular sieve-containing catalyst may precede or follow the reaction zone containing UZM-8 molecular sieve-containing catalyst. Included within the broad aspects of series reaction zones is using both of the FAU and UZM-8 molecular sieves in one or more reaction zones. For example, one reaction zone may contain catalyst comprising both FAU and UZM-8 molecular sieves and another may contain catalyst having only one of the molecular sieves or a different ratio of the molecular sieves. The apparatus may have more than two reaction zones in series. Where a plurality of reaction zones are used, one or more may be of one catalyst composition and at least one of the reaction zones may be of another catalyst composition.

The reaction zones containing different catalyst compositions may be in any suitable sequential order. For instance, the reaction zone containing the FAU catalyst may precede and/or follow a reaction zone containing the UZM-8 catalyst, or the reaction zone containing the UZM-8 catalyst may precede and/or follow a reaction zone containing the FAU catalyst. In one preferred embodiment, the reaction zone in the last sequential position contains catalyst comprising UZM-8 molecular sieve which often provides low co-production of heavies. In another alternative, a combination of parallel and series reaction zones are used wherein either a reaction zone receives the effluents from two or more preceding reaction zones in parallel or the effluent from a reaction zone is provided in aliquot portions to two or more sequential reaction zones in parallel.

In the series configuration, preferably an aliquot portion of the olefin is passed to a first reaction zone and an aliquot portion to a subsequent reaction zone. An aliquot portion of a stream is a fraction of a stream having a similar composition to the total stream from which it is derived. Typically the alkylation reaction is quite rapid under reaction conditions such that most of the olefin is reacted within a short length of the catalyst bed or structure. The amount of catalyst in the bed or structure is generally sufficient such that as the catalyst deactivates, ample catalyst volume remains to effect the sought alkylation. Consequently, it is impractical to operate a reaction zone under conditions where the reaction zone effluent would contain a substantial portion of the olefin. Hence, olefin is introduced into the feeds to each of the reaction zones. The ratio of the olefin fed to each reaction zone will determine the 2-phenylalkane content of the product. Often 25 to 75 mass percent of the olefin is introduced into the reaction zones containing the FAU molecular sieve and the remainder to the reaction zones containing the UZM-8 molecular sieve. To take advantage of the aromatic compound to olefin ratio, it is preferred, but not essential, that the entire aromatic compound feed be fed to the first reaction zone.

With respect to parallel reaction zones, an aliquot portion of the aromatic compound and olefin feed is passed to each of the reaction zones in relative portions sufficient to provide the sought 2-phenylalkane concentration in the phenylalkane product. One or more reaction zones contain catalyst comprising FAU molecular sieve and at least one reaction zone contains UZM-8 molecular sieve. Either reaction zone, if desired, may contain catalyst of the other molecular sieve, but the relative portions of FAU and UZM-8 molecular sieves have to differ between at least two reaction zones for useful control of the 2-phenylalkane concentration. The amount of feed to each reaction zone can be selected to provide the sought 2-phenylalkane concentration. Where one reaction zone contains catalyst only having FAU molecular sieve and the other contains catalyst only having UZM-8 molecular sieve, often 25 to 75 mass percent of the feed is fed to one with the remainder to the other.

With respect to the overall alkylation process, usually the aromatic compound is present in a significant stoichiometric excess to the mono-olefin, e.g., from 2.5:1 up to 50:1 and normally from 6:1 to 35:1, on a molar basis. The processes of this invention are particularly attractive in that low heavies make can be achieved even at lower aromatic compound to olefin mole ratios. In the preferred aspects of the processes of this invention, the aromatic compound to olefin mole ratio is between 6:1 to 25:1, and most preferably between 8:1 to 20:1. The heavies, even at these low ratios, may often be less than 6 mass percent of the phenylalkane product. Co-pending patent application (Attorney Docket No. UOP27674), filed on even date herewith, discloses processes for making alkylbenzenes at low aromatic compound to olefin mole ratios without undue heavies co-production by using small size FAU crystallite catalyst.

The benzene and the olefin are reacted under alkylation conditions in the presence of the catalyst. These alkylation conditions for both catalysts generally include a temperature in the range between 80°C and 200°C, most usually at a temperature not exceeding 175°C. Where different reaction zones are used, each reaction zone may be at different alkylation conditions within these ranges or, preferably, the reaction zones are under common temperature and pressure conditions for ease of operation. The benefits of this invention can still be achieved using common temperature and pressure conditions. Similarly, the reaction zones may provide the same or different space velocities.

Since the alkylation is typically conducted in at least partial liquid phase, and preferably in either an all-liquid phase or at supercritical conditions, pressures must be sufficient to maintain reactants in the liquid phase. The requisite pressure necessarily depends upon the olefin, the aryl compound, and temperature, but normally is in the range of 1300 to 7000 kPa(g), and most usually between 2000 and 3500 kPa(g). Preferably the alkylation conditions do not substantially result in skeletal isomerization of the olefin. For instance, less than 15 mol-%, and preferably less than 10 mol-%, of the olefin, the aliphatic alkyl chain, and any reaction intermediate undergoes skeletal isomerization.

Alkylation of the aromatic compound by the olefins is conducted in a continuous manner. For purposes herein, a catalyst bed is termed a reaction zone whether in the same or a separate vessel from another bed. The number of reaction zones is preferably at least two, and is often three or more. In the processes of this invention 3 or 4 reaction zones can be used for an advantageous combination of performance and capital expense avoidance. Co-pending patent application (Attorney Docket No. 110072), filed on even date herewith discloses a multiple bed alkylation reactor system with interbed cooling to provide an alkylbenzene product having enhanced linearity.

The catalyst may be used as a packed bed or a fluidized bed. The feed to the reaction zone may be passed either upflow or downflow, or even horizontally as in a radial bed reactor. In one desirable variant, olefin-containing feedstock may be fed into several discrete points within the reaction zone, and at each zone the aromatic compound to olefin molar ratio may be greater than 50:1. The total feed mixture, that is, aromatic compound plus the olefin-containing stream, is often passed through the packed bed at a liquid hourly space velocity (LHSV) between 0.3 and 6 hr⁻¹ depending upon, e.g., alkylation temperature and the activity of the catalyst. Where more than one catalyst bed is used in series, the overall LHSV is determined from the LHSV's of each of the beds. The reciprocal of the overall LHSV is the sum of the reciprocals of the LHSV of each of the beds in series.

After passage of the aromatic compound and the olefin through the reaction zone, the effluent is collected and separated into unreacted aromatic compound fraction, which is recycled to the feed end of the reaction zone, and arylalkanes. Where the olefin is obtained by the dehydrogenation of a paraffinic feedstock, any paraffins in the reaction zone effluent are usually separated into a paraffinic fraction, which may be recycled to the dehydrogenation unit. Since the reaction usually goes to at least 98% conversion based on the olefin, little unreacted olefin is recycled with paraffin.

### EXAMPLES

### EXAMPLE 1 (comparative)

Catalyst A is composed of 80% Y zeolite that has been steam dealuminated and acid washed to remove extra framework alumina. These techniques are well known and Y zeolites produced using these techniques are commercially available from a number of companies. The Y zeolite has a unit cell size (UCS) of [24.29 angstroms] 2.429 nm. The zeolite is bound with alumina and extruded into 1.6 mm (1/16 in.) diameter cylinders using ordinary techniques and then calcined at 500°C.

Catalyst A is evaluated in a plug flow reactor at a molar ratio of benzene to olefin ratio of 30:1 under the following conditions: inlet temperature of 130°C and LHSV of 3.75 hr⁻¹.

The olefins are sourced from a commercial plant. The olefin-containing stream contains approximately 12 mass% olefins with the remainder consisting of mostly n-paraffins.

The products of the alkylation are analyzed by gas chromatography (GC) to determine product distribution and by bromine index to determine the amount of unreacted olefin. The olefin conversion is greater than 99.5% and the product distribution is given below in Table 1:

**TABLE 1**

| | |
|---|---|
| Linear monoalkylbenzene | 89 %-mass |
| Non-linear monoalkylbenzene | 8 %-mass |
| Total monoalkylbenzene | 97 %-mass |
| Dialkylbenzene | 3 %-mass |
| 2-phenyl LAB/total monoalkylbenzene | 22 %-mass |

### EXAMPLE 2 (comparative)

Catalyst B is composed of 70% UZM-8 having an atomic ratio of silicon to aluminum of 10.5:1. The UZM-8 is bound with alumina and extruded into 1.6 mm (1/16 in.) cylinders using ordinary techniques and then calcined at 500°C.

Catalyst B is evaluated under the same experimental conditions as catalyst A. The olefin conversion is greater than 99.5% and the product distribution is given below in Table 2:

**TABLE 2**

| | |
|---|---|
| Linear monoalkylbenzene | 94 %-mass |
| Non-linear monoalkylbenzene | 4 %-mass |
| Total monoalkylbenzene | 98 %-mass |
| Dialkylbenzene | 2 %-mass |
| 2-phenyl LAB/total monoalkylbenzene | 47 %-mass |

### EXAMPLE 3

Catalyst C is composed of 60 mass percent Y zeolite of the type used for Catalyst A, 20 mass percent UZM-8 of the type used in Catalyst B, and 20 mass percent alumina. Catalyst C is evaluated under the same experimental conditions as catalyst A. The olefin conversion is greater than 99.5% and the product distribution is given below in Table 3:

**TABLE 3**

| | |
|---|---|
| Linear monoalkylbenzene | 89 %-mass |
| Non-linear monoalkylbenzene | 8 %-mass |
| Total monoalkylbenzene | 97 %-mass |
| Dialkylbenzene | 3 %-mass |
| 2-phenyl LAB/total monoalkylbenzene | 35 %-mass |

The evaluation of Catalyst C indicates that the 2-phenyl content remains relatively constant during the period of use of the catalyst between regenerations and relatively constant with time on stream after the catalyst has passed the initiation period. Catalyst regeneration is done by passing hot benzene over the catalyst.

### EXAMPLE 4

Catalyst D is composed of 68 mass percent Y zeolite of the type used for Catalyst A, 12 mass percent UZM-8 of the type used in Catalyst B and 20 mass percent alumina. Catalyst D is evaluated under the same experimental conditions as catalyst A. The olefin conversion is greater than 99.5% and the product distribution is given below in Table 4:

**TABLE 4**

| | |
|---|---|
| Linear monoalkylbenzene | 89 %-mass |
| Non-linear monoalkylbenzene | 8 %-mass |
| Total monoalkylbenzene | 97 %-mass |
| Dialkylbenzene | 3 %-mass |
| 2-phenyl LAB/total monoalkylbenzene | 29 %-mass |

The evaluation of Catalyst D indicates that the 2-phenyl content remains relatively constant during the period of use of the catalyst between regenerations and relatively constant with time on stream after the catalyst has passed the initiation period.

### EXAMPLE 5

A uniform mixture of 72 mass percent Catalyst A and 28 mass percent Catalyst B is prepared and is evaluated under the same experimental conditions as Catalyst C. Substantially the same performance is achieved as with Catalyst C.

## Claims

1. A process for producing a phenylalkane product comprising contacting at least one aromatic compound and at least one acyclic mono-olefin with solid catalyst under alkylation conditions wherein at least a portion of said aromatic compound and at least a portion of said mono-olefin are contacted with a catalyst comprising acidic FAU molecular sieve and at least a portion of said aromatic compound and at least a portion of said mono-olefin are contacted with a catalyst comprising UZM-8 molecular sieve to provide a phenylalkane product having a 2-phenyl content of between 25 and 40 mass percent, wherein the aromatic compound is benzene.

2. The process of claim 1 wherein the catalyst comprising FAU molecular sieve and the catalyst comprising UZM-8 molecular sieve are in distinct reaction zones.

3. The process of claim 1 or 2 wherein the portion of said aromatic compound and mono-olefin contacted with the catalyst comprising FAU molecular sieve and the portion of said aromatic compound and mono-olefin contacted with the catalyst comprising UZM-8 molecular sieve are selected to provide a phenylalkane product having a desired 2-phenyl content.

4. The process of claim 1 or 2 wherein the FAU molecular sieve is zeolite Y, the mono-olefin has from 8 to 16 carbon atoms per molecule, and the 2-phenylalkane content is between 26 and 36 mass percent.

5. The process of any of claims 1 to 4 wherein the catalyst comprising FAU molecular sieve and the catalyst comprising UZM-8 molecular sieve are in the same reaction zone.

6. The process of claim 5 wherein the relative amounts of the FAU molecular sieve and the UZM-8 molecular sieve in the reaction zone are selected to provide a phenylalkane product having a desired 2-phenyl content.

7. The process of claim 6 wherein the FAU molecular sieve is zeolite Y, the mono-olefin has from 8 to 16 carbon atoms per molecule, and the 2-phenylalkane content is between 26 and 36 mass percent.

8. The process of claim 5 wherein the molar ratio of aromatic compound to mono-olefin is between 6:1 to 25:1.

9. The process of claim 8 wherein the phenylalkane product has a linearity of at least 89 mass percent.

## Patentansprüche

1. Verfahren zur Herstellung eines Phenylalkanprodukts, bei dem man mindestens eine aromatische Verbindung und mindestens ein acyclisches Monoolefin unter Alkylierungsbedingungen mit einem festen Katalysator in Berührung bringt, wobei man mindestens einen Teil der aromatischen Verbindung und mindestens einen Teil des Monoolefins mit einem Katalysator, der saures FAU-Molsieb umfasst, in Berührung bringt und mindestens einen Teil der aromatischen Verbindung und mindestens einen Teil des Monoolefins mit einem Katalysator, der UZM-8-Molsieb umfasst, in Berührung bringt, wobei man ein Phenylalkanprodukt mit einem 2-Phenylgehalt zwischen 25 und 40 Massenprozent erhält, wobei es sich bei der aromatischen Verbindung um Benzol handelt.

2. Verfahren nach Anspruch 1, bei dem der Katalysator, der FAU-Molsieb umfasst, und der Katalysator, der UZM-8-Molsieb umfasst, in verschiedenen Reaktionszonen vorliegen.

3. Verfahren nach Anspruch 1 oder 2, bei dem man den Teil der aromatischen Verbindung und des Monoolefins, der mit dem Katalysator, der FAU-Molsieb umfasst, in Berührung gebracht wird, und den Teil der aromatischen Verbindung und des Monoolefins, der mit dem Katalysator, der UZM-8-Molsieb umfasst, in Berührung gebracht wird, so wählt, dass man ein Phenylalkanprodukt mit einem gewünschten 2-Phenylgehalt erhält.

4. Verfahren nach Anspruch 1 oder 2, bei dem es sich bei dem FAU-Molsieb um Y-Zeolith handelt, das Monoolefin 8 bis 16 Kohlenstoffatome pro Molekül aufweist und der 2-Phenylalkangehalt zwischen 26 und 36 Massenprozent liegt.

5. Verfahren nach einem der Ansprüche 1 bis 4, bei dem der Katalysator, der FAU-Molsieb umfasst, und der Katalysator, der UZM-8-Molsieb umfasst, in derselben Reaktionszone vorliegen.

6. Verfahren nach Anspruch 5, bei dem man die relativen Mengen des FAU-Molsiebs und des UZM-8-Molsiebs in der Reaktionszone so wählt, dass man ein Phenylalkanprodukt mit einem gewünschten 2-Phenylgehalt erhält.

7. Verfahren nach Anspruch 6, bei dem es sich bei dem FAU-Molsieb um Y-Zeolith handelt, das Monoolefin 8 bis 16 Kohlenstoffatome pro Molekül aufweist und der 2-Phenylalkangehalt zwischen 26 und 36 Massenprozent liegt.

8. Verfahren nach Anspruch 5, bei dem das Molverhältnis von aromatischer Verbindung zu Mono-olefin zwischen 6:1 und 25:1 liegt.

9. Verfahren nach Anspruch 8, bei dem das Phenylalkanprodukt eine Linearität von mindestens 89 Massenprozent aufweist.

## Revendications

1. Procédé de production d'un produit de phénylalcane comprenant la mise en contact d'au moins un composé aromatique et d'au moins une mono-oléfine acyclique avec un catalyseur solide dans des conditions d'alkylation, au moins une partie dudit composé aromatique et au moins une partie de ladite mono-oléfine étant mises en contact avec un catalyseur comprenant un tamis moléculaire FAU acide et au moins une partie dudit composé aromatique et une partie de ladite mono-oléfine étant mises en contact avec un catalyseur comprenant un tamis moléculaire UZM-8 pour donner un produit de phénylalcane ayant une teneur en 2-phényle comprise entre 25 et 40 pour cent en masse, le composé aromatique étant le benzène.

2. Procédé selon la revendication 1, **caractérisé en ce que** le catalyseur comprenant le tamis moléculaire FAU et le catalyseur comprenant le tamis moléculaire UZM-8 sont dans des zones de réaction distinctes.

3. Procédé selon la revendication 1 ou 2, **caractérisé en ce que** la partie dudit composé aromatique et de ladite mono-oléfine mise en contact avec le catalyseur comprenant le tamis moléculaire FAU et la partie dudit composé aromatique et de la ladite mono-oléfine mise en contact avec le catalyseur comprenant le tamis moléculaire UZM-8 sont choisies pour donner un produit de phénylalcane ayant une teneur en 2-phényle souhaitée.

4. Procédé selon la revendication 1 ou 2, **caractérisé en ce que** le tamis moléculaire FAU est une zéolite Y, la mono-oléfine a de 8 à 16 atomes de carbone par molécule, et la teneur en 2-phénylalcane est comprise entre 26 et 36 pour cent en masse.

5. Procédé selon l'une quelconque des revendications 1 à 4, **caractérisé en ce que** le catalyseur comprenant le tamis moléculaire FAU et le catalyseur comprenant le tamis moléculaire UZM-8 sont dans la même zone de réaction.

6. Procédé selon la revendication 5, **caractérisé en ce que** les quantités relatives du tamis moléculaire FAU et du tamis moléculaire UZM-8 dans la zone de réaction sont choisies pour donner un produit de phénylalcane ayant une teneur en 2-phényle souhaitée.

7. Procédé selon la revendication 6, **caractérisé en ce que** le tamis moléculaire FAU est la zéolite Y, la mono-oléfine a de 8 à 16 atomes de carbone par molécule et la teneur en 2-phénylalcane est comprise entre 26 et 36 pour cent en masse.

8. Procédé selon la revendication 5, **caractérisé en ce que** le rapport molaire entre le composé aromatique et la mono-oléfine est compris entre 6:1 à 25:1.

9. Procédé selon la revendication 8, **caractérisé en ce que** le produit de phénylalcane a une linéarité d'au moins 89 pour cent en masse.
